# EUROPÄISCHE PATENTSCHRIFT

(11) **EP 4 292 568 B1**
(45) Veröffentlichungstag und Bekanntmachung des Hinweises auf die Patenterteilung: **06.08.2025**
(21) Anmeldenummer: 23175656.0
(22) Anmeldetag: 26.05.2023
(51) Int. Cl.: A61F 2/18, A61L 27/06, A61L 27/14

(54) **LÄNGENVERSTELLBARE GEHÖRKNÖCHELCHENPROTHESE MIT IN-SITU-ELONGATION AUS DER KOPFPLATTE HERAUS**
LENGTH-ADJUSTABLE OSSICULAR PROSTHESIS WITH IN-SITU ELONGATION OUT OF HEAD PLATE
PROTHÈSE D'OSSELETS D'OREILLE À LONGUEUR RÉGLABLE DOTÉE D'UNE ALLONGEMENT IN SITU DEPUIS LA PLAQUE DE TÊTE

(30) Priorität: 15.06.2022 DE 202022103367 U
(43) Veröffentlichungstag der Anmeldung: 20.12.2023
(73) Patentinhaber: Heinz Kurz GmbH, 72144 Dusslingen (DE)
(72) Erfinder: STIEGELE, Thomas, 72585 Riederich (DE); SANDER, Alexander, 79822 Titisee-Neustadt (DE); MERTENS, Matthias, 24568 Kaltenkirchen (DE); GÄCKLE, Markus, 75378 Bad Liebenzell (DE)
(74) Vertreter: Kohler Schmid Möbus Patentanwälte

(56) Entgegenhaltungen:
- WO-A1-2018/052866
- DE-A1- 19 845 906
- SU-A1- 957 894
- US-B2- 10 687 937

## Beschreibung

Die Erfindung ist in Anspruch 1 definiert und betrifft eine Gehörknöchelchenprothese, die mindestens ein Glied oder Teile eines Gliedes der Gehörknöchelchenkette ersetzt oder überbrückt, wobei die Gehörknöchelchenprothese an ihrem einen Ende ein als Kopfplatte zur mechanischen Anlage am Trommelfell und/oder am Hammergriff ausgebildetes erstes Befestigungselement und an ihrem anderen Ende ein zweites Befestigungselement zur mechanischen Verbindung mit einem Glied oder Teilen eines Gliedes der Gehörknöchelchen-Kette oder mit dem Innenohr sowie ein entlang einer Längsachse die beiden Befestigungselemente Schall leitend miteinander verbindendes Verbindungselement umfasst, und wobei das Verbindungselement Stegelemente aufweist, die -zumindest abschnittsweise- radial von der Längsachse weg nach außen mehr oder weniger abgespreizt werden können und dabei die axiale Länge der Gehörknöchelchenprothese mehr oder weniger verkürzen, wobei die Stegelemente einenends direkt in Ankoppelbereiche des ersten Befestigungselements innerhalb der Kopfplattenebene münden und mit diesen beweglich, aber unlösbar verbunden sind, wobei sämtliche Stegelemente anderenends direkt in ein Ankoppelelement münden und mit diesem ebenfalls beweglich, aber unlösbar verbunden sind, wobei das Ankoppelelement seinerseits anderenends mit dem zweiten Befestigungselement starr verbunden ist, wobei die Stegelemente derart ausgebildet sind, dass sie in einem im menschlichen Mittelohr eingesetzten in-situ-Zustand der Gehörknöchelchenprothese bei Einleitung einer Kraft auf die Stegelemente mit Kraftkomponente parallel zur Längsachse in Richtung vom ersten Befestigungselement zum zweiten Befestigungselement in situ jeweils abschnittsweise eine radial weiter von der Längsachse liegende Position einnehmen und damit die axiale Funktionslänge zwischen dem ersten Befestigungselement und dem zweiten Befestigungselement in situ verkürzen, wobei die Stegelemente bei Einleitung einer Kraft mit Kraftkomponente antiparallel zur Längsachse in Richtung vom zweiten Befestigungselement zum ersten Befestigungselement in situ jeweils abschnittsweise eine radial näher an der Längsachse liegende Position einnehmen und damit die axiale Funktionslänge zwischen dem ersten Befestigungselement und dem zweiten Befestigungselement in situ vergrößern, und wobei die Stegelemente ohne Einwirkung einer Kraft ihre jeweils eingestellte radiale Position zur Längsachse in situ beibehalten.

Eine derartige Vorrichtung ist bekannt aus der DE 198 45 906 A1 (=Referenz [0]).

Eine ähnliche Vorrichtung zeigt auch schon die SU 957 894 A1 (=Referenz [00]).

### Hintergrund der Erfindung

Gehörknöchelchenprothesen werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell beziehungsweise am Hammergriff anliegt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten GehörknöchelchenProthesen die Schallleitung zwischen dem Trommelfell und dem Innenohr nur begrenzt ermöglicht, weil sie die natürlichen anatomischen Ausbildungen der Gehörknöchelchenkette nur sehr eingeschränkt ersetzen können. (Siehe zum Beispiel DE 42 10 235 C1; EP 0 809 982 B1; US 6,387,128 B1 (=Referenz [2])).

Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Bei einer steifen Verbindung zwischen Kopfplatte und Schaft kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration oder Extrusion durch das Trommelfell die Folge wäre. Aus diesem Grund ist es sehr hilfreich, wenn die Prothese eine gewisse post-operative Mobilität aufweist, so dass sich die Kopfplatte postoperativ selbstständig der Position des Trommelfells angleichen kann.

Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Gehörknöchelchenprothesen nicht starr ausgebildet sind, sondern eine gewisse Flexibilität oder Variabilität aufweisen.

Um eine solche Flexibilität/Variabilität zu erreichen sind verschiedene Befestigungs- und Ankopplungsvorrichtungen für Gehörknöchelchen, die elastische Teile und/oder Gelenke aufweisen, bekannt. Eine solche gelenkige Verbindung zwischen einem an der Steigbügelfußplatte montierbaren Befestigungselement und dem länglichen Schaft ist beispielsweise in der EP 1 181 907 B1 (=Referenz [3]) beschrieben und wird von der Anmelderin unter dem Markennamen "Ball-Joint" angeboten.

Eine weitere, verschiedentlich auftretende Komplikation entsteht durch eine Unterbelüftung des Mittelohrraumes und damit einhergehende Entzündungen, Tumorbildungen, Verwachsungen im Trommelfellbereich sowie Versteifungen desselben. Beispielsweise bei einer Dysfunktion der Eustachischen Röhre kann es im Mittelohr zu einem Unterdruck kommen, der eine Auskrempung bzw. Ausstülpung (sogenannte Retraktion) des Trommelfells und in der Folge eine Verwachsung etwa mit dem Steigbügel bewirken kann. Um dem entgegen zu wirken und postoperativen Bewegungen des Trommelfells folgen zu können, werden die Kopfplatten bei bekannten Gehörknöchelchenprothesen verkippbar relativ zu dem Verbindungselement gestaltet, das die Kopfplatte mit dem zweiten Befestigungselement verbindet uns meistens als länglicher Schaft ausgeführt ist. Eine solche in sich starre, aber gegenüber dem Verbindungselement verkippbare Kopfplatte ist u.a. beschrieben in der US 2004/0162614 A1 (=Referenz [4]).

Während Referenz [3] eine Gehörknöchelchenprothese, bei welcher die Kopfplatte ein eingebautes, mit dem Verbindungsschaft der beiden Befestigungselemente verbundenes Kugelgelenk aufweist, offenbart EP 1 833 424 B1 (=Referenz [5]) sogar einen als Kugelkette ausgebildeten Verbindungsschaft. Dadurch kann die endgültige axiale Länge der Prothese zumindest abgestuft durch die Auswahl einer bestimmten Anzahl an Kugeln in der Kette und Abschneiden der überzähligen Kugeln festgelegt werden.

Nachteilig bei diesen bekannten Gehörknöchelchenprothesen ist allerdings, dass durch die starre Verkippung der Kopfplatte bei lokalen Medialbewegungen des Trommelfells gleichzeitig die gegenüber liegende Seite der Kopfplatte lateral nach außen bewegt wird, wodurch Druckspitzen auf das Trommelfell erzeugt werden.

Die EP 1 972 307 B1 (=Referenz [6]) offenbart eine Gehörknöchelchenprothese mit hochflexibel gestalteter Kopfplatte, bei welcher einerseits die Vorteile der Prothese gemäß der oben genannten Referenz [5], andererseits aber auch die Vorteile der in Referenz [4] beschriebenen Prothesen erhalten bleiben, wobei jedoch die gemeinsamen Nachteile einer starren Verkippung der Kopfplatte vermieden werden.

Problematisch bei der Gehörknöchelchenprothese nach Referenz [6] ist allerdings ihre Einführbarkeit ins Mittelohr des Patienten, weil insbesondere in radialer Richtung bezüglich ihrer Längsachse die Kopfplatte erheblich seitlich ausragt und nur mittels einer großen künstlichen Öffnung operativ durch den Trommelfell-Bereich in das Mittelohr eingesetzt werden kann. Diese große Öffnung wächst dann post-operativ natürlich auch schwerer wieder zu und hinterlässt zudem entsprechend große Narben.

Zwar umfasst die Kopfplatte bei Referenz [6] flexible Stegelemente, welche innerhalb der Kopfplatten-Ebene verlaufen und einen radial äußeren Ringbereich der Kopfplatte mit einem radial in der Mitte der Kopfplatte angeordneten zentralen Ankoppelbereich verbinden. Diese Stegelemente sind -zusammen mit dem radial äußeren Ringbereich und dem zentralen Ankoppelbereich- fester Bestandteil der in Referenz [6] offenbarten Kopfplatte. Sie sind geometrisch so gestaltet, dass sie bei lokalen Medialbewegungen des Trommelfells einer solchen Medialbewegung -ebenfalls lokal- folgen, jedoch die Bewegung nicht an entfernte Bereiche der Kopfplatte weitergeben.

Für eine etwaige radiale Stauchung der Kopfplatte innerhalb der Kopfplatten-Ebene und damit einer radialen Verkleinerung des Kopfplatten-Durchmesser, gegebenenfalls zum Zwecke einer erleichterten Durchführung durch das Trommelfell, sind die Stegelemente aber nicht ausgebildet, sondern wären für eine derartige Anwendung ungeeignet. Dies ist insbesondere deswegen so, weil der radial äußere Ringbereich starr ausgeführt ist und daher dem Versuch einer solchen radialen Stauchung nicht folgen oder sich höchstens unkontrollierbar verbiegen würde.

Auch für eine Längenveränderung in Richtung der z-Achse ist die in Referenz [6] beschriebene Gehörknöchelchenprothese absolut ungeeignet, da das Verbindungselement, welches längs der z-Achse verläuft und die Kopfplatte mit dem zweiten Befestigungselement starr verbindet, zwar Schall-übertragend, aber als solches steif ausgebildet ist. Eine Flexibilität des Länge des Verbindungselements in z-Richtung ist bei der Gehörknöchelchenprothese nach Referenz [6] ausgeschlossen und auch gar nicht gewünscht.

Die EP 3 311 773 B1 (=Referenz [7]) beschreibt demgegenüber eine Gehörknöchelchenprothese mit regenschirmartig faltbarer Kopfplatte, mittels welcher die Prothese nahezu minimal-invasiv durch eine kleine Öffnung im Trommelfell operativ ins Mittelohr einsetzbar ist.

Ein weiteres wichtiges Thema bei der Implantation von Gehörknöchelchenprothesen ist die Einstellung der korrekten, an die individuellen Gegebenheiten und Geometrieverhältnisse im Mittelohr des Patienten optimal angepassten axialen Länge der Prothese.

Bereits die oben diskutierte Referenz [5] schlägt dazu eine Kugelkette mit abtrennbaren Endkugeln vor. Dies ermöglicht aber leider keine kontinuierliche, sondern lediglich eine stufenweise Längeneinstellung.

Eine längenvariable Gehörknöchelchenprothese mit einem im Verbindungselement zwischen dem ersten und dem zweiten Befestigungselement eingebauten Verschiebemechanismus zur stufenlosen Längeneinstellung ist in der DE 10 2007 041 539 B4 (=Referenz [8]) beschrieben.

Anstelle einer solchen -in der Herstellung relativ aufwändigen-Verschiebemechanik schlägt die EP 2 238 946 B1 (=Referenz [9]) eine längenvariable Gehörknöchelchenprothese vor, bei welcher im Verbindungselement eine Ziehharmonika-artige Struktur eingebaut ist. Die axiale Länge der Gehörknöchelchenprothese kann dann durch eine axiale Stauchung dieser Struktur verkürzt und durch ein Auseinanderziehen derselben vergrößert werden.

In der EP 2 601 909 B1 (=Referenz [10]) wiederum ist zur axialen Längeneinstellung der Gehörknöchelchenprothese eine Verschiebemechanik im Verbindungselement vorgesehen, welche ein Aufnahmeteil und einen das Aufnahmeteil mit zwei Schenkeln klammerartig umgreifendes Einschiebeteil umfasst, wobei das Aufnahmeteil und das Einschiebeteil relativ zueinander in axialer Richtung des Verbindungselements verfahrbar sind.

Eine Laser-aktivierbare längenvariable Gehörknöchelchenprothese schließlich ist in der EP 3 130 315 B1 (=Referenz [11]) offenbart. Darin wird vorgeschlagen, das Verbindungselement mit streckbaren und/oder stauchbaren, schlaufenartig gefalteten Teilsträngen aus einem Material mit Formgedächtnis aufzubauen. Mit den Schlaufen dieser Teilstränge sind Aktivierungsflächen wärmeleitend verbunden, welche durch Hitzeeinwirkung eine thermische Aktivierung und damit eine Verformung der Schlaufen bewirken können. Auf diese Weise kann die axiale Länge der Gehörknöchelchenprothese verändert und nach Wunsch eingestellt werden.

Die US 10,687,937 B2 (=Referenz [1]) schließlich offenbart eine Gehörknöchelchenprothese, die mindestens ein Glied oder Teile eines Gliedes der Gehörknöchelchenkette ersetzt oder überbrückt, wobei die Gehörknöchelchenprothese an ihrem einen Ende ein als Kopfplatte zur mechanischen Anlage am Trommelfell und/oder am Hammergriff ausgebildetes erstes Befestigungselement und an ihrem anderen Ende ein zweites Befestigungselement zur mechanischen Verbindung mit einem Glied oder Teilen eines Gliedes der Gehörknöchelchen-Kette oder mit dem Innenohr sowie ein entlang einer Längsachse die beiden Befestigungselemente Schall leitend miteinander verbindendes Verbindungselement umfasst, und wobei das Verbindungselement Stegelemente aufweist, die -zumindest abschnittsweise- radial von der Längsachse weg nach außen mehr oder weniger abgespreizt werden können und dabei die axiale Länge der Gehörknöchelchenprothese mehr oder weniger verkürzen. Insbesondere wird vorgeschlagen, im Verbindungselement streifenartige Stegelemente vorzusehen, die radial von der Längsachse weg nach außen abgespreizt werden können und dabei die axiale Länge des Verbindungselements und damit die axiale Länge der Gehörknöchelchenprothese verkürzen. Die Spreizung der Stegelemente erfolgt von der Seite her, also in radialer Richtung bezüglich des Verbindungselements. Eine Vergrößerung der axialen Länge ist bei diesem Wirkmechanismus allerdings nicht vorgesehen. Ohne zusätzliche Maßnahmen ist diese Längeneinstellung auch nur vor dem Einsetzen der Prothese ins Mittelohr des Patienten möglich, nicht jedoch in situ im implantierten Zustand.

### Aufgabe der Erfindung

Aufgabe der vorliegenden Erfindung ist es demgegenüber, eine gattungsgemäße längenverstellbare Gehörknöchelchenprothese der eingangs definierten Art mit möglichst einfachen technischen Mitteln dahingehend zu verbessern, dass unaufwändig und kostengünstig eine Längenverstellung in situ bei bereits platzierter Prothese erfolgen kann, dass die Längenverstellung der Prothese auch von oben her möglich ist, dass dabei eine gleichmäßige und symmetrische Elongation der Prothese sichergestellt wird, und dass eine Anwendung der Erfindung auch etwa bei Partialprothesen mit geringer axialer Bauhöhe und/oder geringer funktionaler Länge oder ermöglicht wird.

### Kurze Beschreibung der Erfindung

Erfindungsgemäß wird diese Aufgabe auf ebenso überraschend einfache wie wirkungsvolle Art und Weise dadurch gelöst, dass die Ankoppelbereiche, die Stegelemente sowie das Ankoppelelement vor dem erstmaligen Einleiten einer Kraft auf die Stegelemente mit Kraftkomponente parallel oder antiparallel zur Längsachse flach innerhalb der Kopfplattenebene des ersten Befestigungselements angeordnet sind; dass die Stegelemente zwischen ihrem jeweiligen Ankoppelbereich und dem Ankoppelelement kurvenförmig und/oder mäanderförmig und/oder im Zickzack verlaufen; dass das Ankoppelelement in seiner Ausgangsposition innerhalb der Kopfplattenebene zentrisch im ersten Befestigungselements angeordnet ist; dass radial äußere, freie Endabschnitte der Stegelemente atraumatische, nicht-spitzzulaufende, bezüglich der Längsachse radial äußere, quer zur Richtung der Längsachse ausgedehnte, freie Endkanten aufweisen oder als atraumatische Flächen ausgebildet sind; und dass Arretierungseinrichtungen vorhanden sind, welche beim Einleiten einer axialen Kraft auf die Stegelemente bei einer oder mehreren axialen Längen des Verbindungselements jeweils einen mechanischen Widerstand hervorrufen.

Damit wird ein besonders kompakter Aufbau der erfindungsgemäßen Gehörknöchelchenprothese ermöglicht.

Dadurch können auf relativ simple Weise die Vorteile der oben beschriebenen gattungsgemäßen Gehörknöchelchenprothese, wie sie in Referenzen [0] oder [00] offenbart ist, genutzt werden, wobei jedoch die Längenänderung bei bereits im Mittelohr platzierter Prothese erfolgen kann, was z.B. bei der Prothese bei Referenz [1] nicht oder nur mit ganz erheblichen Änderungen möglich wäre.

Durch die Möglichkeit zur Umwandlung einer mechanischen Bewegung innerhalb der Fläche der Kopfplatte in eine axiale Längenverstellung, wobei die axiale Bewegung letztlich immer aus der Kopfplattenebene herausführen wird, kann bei der erfindungsgemäßen Gehörknöchelchenprothese eine feinjustierbare Längeneinstellung auch erst nach dem Einsetzen ins Mittelohr, also in situ vorgenommen werden. Die Längenänderung, also die Veränderung des Abstands zwischen der Kopfplatte und dem zweiten Befestigungselement am anderen Ende der Prothese, erfolgt dabei durch eine Expansion aus der Kopfplattenebene heraus in die Tiefe (oder vice versa).

Der erfindungsgemäße Mechanismus ist automatisch innerhalb der Kopfplatte geführt und stellt daher auch ohne zusätzliche Maßnahmen eine gleichmäßige und vor allem symmetrische Elongation sicher. Die Ansätze der im Stand der Technik, insbesondere in den Referenzen [0], [00] und [1] beschriebenen Lösungen erfordern im Gegensatz dazu, dass der Anwender an beiden Seiten die Prothese manipulieren muss um eine symmetrische Längenänderung hervorzurufen.

Da der Mechanismus zur Längenverstellbarkeit in der Kopfplatte integriert und mit dieser verbaut ist, kann die erfindungsgemäße Gehörknöchelchenprothese auch mit einer sehr geringen Bauhöhe hergestellt werden.

Außerdem kann der erfindungsgemäße Mechanismus von oben her in der Kopfplatte bedient werden. Das ist ebenfalls ein wesentlicher Unterschied zum Stand der Technik. So ermöglichen etwa die Prothesen gemäß den Referenzen [0], [00] und [1] nur eine umständliche und relativ viel Raum erfordernde Manipulation von der Seite her. Der erfindungsgemäße Ansatz hingegen umfasst einen äußerst platzsparenden Aufbau und ist auch für endoskopische Zugänge geeignet.

Durch den nicht geradlinigen, sondern kurvenförmigen Verlauf verstärkt sich die erwünschte Wirkung einer lokalen Flexibilität der Kopfplatte und ein lediglich lokal begrenztes Ausweichen bei kleineren Medialbewegungen des Trommelfells. Zudem kann dadurch die Kopfplatte einer eventuellen post-operativen Veränderung des Trommelfells leichter folgen.

Der erfindungsgemäß Mechanismus wird mit Hilfe eines zentrischen Ansatzpunkts aus der Kopfplatte "herausgedrückt". Die axiale Länge der Prothese expandiert durch plastische Verformung.

Radial äußere, freie Endabschnitte der Stegelemente sind als geschlossene Flächen oder Ringflächen, insbesondere kreisringförmig oder elliptisch, ausgebildet. So können bei einem in der Heilungsphase auftretenden Verkippungsmoment, welches eine erhöhte Kraftwirkung auf die äußeren Endabschnitte zur Folge hat, die Flächenpressung und die Extrusionsgefahr reduziert werden.

Die Verstell-Mechanik ist mit Arretierungspunkten versehen, um eine definierte axiale Länge der Prothese zu erreichen und diese Länge zu fixieren.

Durch den geringen Krafteintrag, wird sichergestellt, dass umliegende Mittelohrstrukturen nicht beschädigt werden.

Durch die Arretierungseinrichtungen, werden optimale Sichtverhältnisse während des Eingriffs ermöglicht.

Eine Wiederaufnahme des Implantats ist während des Eingriffs möglich, um eine Neujustierung zu gewährleisten.

### Bevorzugte Ausführungsformen der Erfindung

Besonders bevorzugte Ausführungsformen der erfindungsgemäßen Gehörknöchelchenprothese zeichnen sich dadurch aus, dass die Stegelemente derart ausgebildet sind, dass ihre jeweils eingestellte radiale Position zur Längsachse in situ durch Einleiten einer entsprechenden Kraft reversibel veränderbar ist.

Insbesondere kann damit die Bewegung der Prothesenteile beim Einstellvorgang reversibel gestaltet und die Prothesenlänge also nicht nur vergrößert, sondern auch wieder verkürzt werden.

Durch den geringen Krafteintrag wird sichergestellt, dass umliegende Mittelohrstrukturen nicht beschädigt werden.

Durch das ausgeklügelte erfindungsgemäße Design der Kopfplatte werden optimale Sichtverhältnisse während des Eingriffs ermöglicht. Durch die Leichtbauweise wird eine optimale Schallübertragung vor allem im Hochtonbereich ermöglicht.

Durch das erfindungsgemäße Kopfplatten Design wurde eine optionale Kopplung an den Hammergriff (Malleus) ermöglicht.

Das erfindungsgemäße Design wurde so entwickelt, dass keine Toträume vorhanden sind. Daher können sich keine Bio-Filme an dem Implantat bilden und Implantat-assoziierte Infektionen ausbilden

Bevorzugt sind auch Ausführungsformen, bei welchen die innerhalb der Kopfplattenebene des ersten Befestigungselements angeordneten Ankoppelbereiche geometrisch so gestaltet sind, dass über sie die Einleitung einer Kraft auf die Stegelemente mit Kraftkomponente parallel oder antiparallel zur Längsachse mittels eines Einstellwerkzeugs in situ erfolgen kann.

Die in-situ Längenverstellung der Gehörknöchelchenprothese lässt sich dadurch erreichen, dass die Struktur innerhalb der Kopfplatte manipuliert wird. Drückt man die Struktur mit einem Instrument zusammen, verlängert sich die Prothese. Spreizt man die Struktur, verkürzt sich die Länge. Das Einstellwerkzeug dient dabei als Implantationshilfe und insbesondere zur Bedienung des Mechanismus zur Längeneinstellung. Es kann als minimalinvasives, insbesondere endoskopisches, Instrument, vorzugsweise Pinzetten-artig oder Zangen-artig, ausgebildet sein.

Eine Längenänderung der Gehörknöchelchenprothese kann prinzipiell durch plastische Verformung der Verbindungsstreben und/oder durch integrierte Gelenke erfolgen.

Daher sind Ausführungsformen der Erfindung von Vorteil, bei denen die Verbindungsstellen der Stegelemente mit den Ankoppelbereichen des ersten Befestigungselements sowie die Verbindungsstellen der Stegelemente mit dem Ankoppelelement jeweils als mechanische Gelenke oder Biegestellen ausgebildet sind.

Eine erste Klasse von Ausführungsformen der erfindungsgemäßen Gehörknöchelchenprothese zeichnet sich dadurch aus, dass die Stegelemente zumindest abschnittsweise, vorzugsweise vollständig mechanisch starr ausgebildet sind.

Bei vorteilhaften Weiterbildungen dieser ersten Klasse von Ausführungsformen sind zwischen den mechanisch starren Abschnitten der Stegelemente integrierte mechanische Gelenke oder Biegestellen ausgebildet.

Damit kann eine gewisse Flexibilität bzw. Variabilität der Prothese erreicht werden, wie sie an sich in der Referenz [3] beschrieben ist. Vorteilhaft im Hinblick auf eine besonders hohe postoperative Beweglichkeit der Prothese sind Weiterbildungen, bei denen eine Vielzahl von aneinander angrenzenden weiteren Drehelementen, vorzugsweise eine Kugelgelenkkette, verwendet werden.

Bei einer zweiten Klasse von dazu alternativen Ausführungsformen der Erfindung sind die Stegelemente zumindest abschnittsweise aus einem plastischen, flexiblen Material ausgebildet, wobei das plastische, flexible Material der Stegelemente insbesondere eine Elastizität ≥ 1%, vorzugsweise eine Elastizität ≥ 2%, aufweist.

Wenn die Stegelemente aus flexiblem Material bestehen, kann der Platz und der technische Aufwand für die mechanischen Drehgelenke sind eingespart werden. Besitzt das Material der Stegelemente eine Flexibilität von >1% kann die Prothese besonders "eng" gebaut werden, wobei auch sichergestellt wird, dass die Prothese kleinsten "topografischen" Änderungen des Trommelfells folgen kann.

Eine erste Gruppe von Weiterbildungen dieser zweiten Klasse von Ausführungsformen, bei welchen das plastische, flexible Material der Stegelemente hochelastisches Material, vorzugsweise amorphes Metall, insbesondere basierend auf Nickel, Eisen, Kobalt oder Zirkonium, und/oder eine Nickel-Titan-Legierung und/oder Memory-Metall, enthält. Die oben genannten Eigenschaften der Flexibilität bei gleichzeitiger für Schallleitung guter Steifigkeit können mit den erwähnten Materialien optimal erreicht werden.

Insbesondere das Material Titan weist neben seiner Festigkeit und ausgezeichneten Schallleitungseigenschaften bekanntermaßen auch eine hervorragende Biokompatibilität am menschlichen Mittelohr auf. Vorteilhaft im Hinblick auf eine postoperative Lageanpassung sind Ausführungsformen der Erfindung, bei denen die Prothese oder Teile davon, insbesondere eines der Befestigungselemente, aus einem Material mit Formgedächtnis (=memory effect) oder superelastischen Eigenschaften, vorzugsweise aus Nitinol hergestellt sind, was per se beispielsweise aus der EP 1 961 400 B1 (=Referenz [12]) bekannt ist.

Bei einer zweiten Gruppe von Weiterbildungen kann das plastische, flexible Material der Stegelemente aber auch hochelastischen Kunststoff, insbesondere hochfestes elastisches Polymer, und/oder elastische Keramik enthalten.

Diese Werkstoffe ermöglichen, bei hoher Flexibilität und guter Steifigkeit, aber bei geringerer Dichte ideale Voraussetzungen für eine gute Schallübertragung.

Außerdem kann bei Ausführungsformen auch noch dadurch eine Verbesserung erreicht werden, dass das Ankoppelelement ringförmig aufgebaut ist.

Bei weiteren Ausgestaltungen der Erfindung ist das zweite Befestigungselement als Platte, insbesondere als gebogene Platte, als Hülse, als Schlinge, als geschlossene Glocke, insbesondere in Form eines Hohlzylinders, als einfach oder mehrfach geschlitzte Glocke oder als Clip zur mechanischen Verbindung mit einem weiteren Glied der Gehörknöchelchenkette ausgebildet. Alternative Ausgestaltungen können vorsehen, dass die Gehörknöchelchenprothese an ihrem das zweite Befestigungselement tragenden Ende mittels Perforation der Steigbügelfußplatte (=Stapedektomie bzw. Stapedotomie) und/oder mittels Eröffnung der menschlichen Hörschnecke (=Cochleotomie) direkt an das Innenohr angekoppelt ist, insbesondere über einen Kolben.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager" dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein zeitlich lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausgestaltung der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen. Die Kopfplatte der erfindungsgemäßen Gehörknöchelchenprothese sollte grundsätzlich eine wachstumsfördernde Beschichtung, ein direkt ins Innenohr führendes, etwa in Form eines Kolbens ausgebildetes zweites Befestigungselement hingegen eher eine wachstumshemmende Beschichtung aufweisen.

Alternativ oder ergänzend können bei weiteren Ausführungsformen Teile der erfindungsgemäßen Gehörknöchelchenprothese aus einem Keramikmaterial hergestellt sein. Möglich sind aber auch Ausführungsformen der Erfindung, bei denen die Prothese oder Teile davon aus biokompatiblen Kunststoffen, insbesondere Silikon, Polytetrafluorethylen (PTFE) oder Faserverbund-werkstoffen hergestellt sind.

Mit diesen Materialien können postoperative Abstoßungsreaktionen in den meisten Fällen ebenfalls verhindert werden.

In den Rahmen der vorliegenden Erfindung fällt auch ein System umfassend eine erfindungsgemäß aufgebaute Gehörknöchelchenprothese der oben beschriebenen Art sowie ein Einstellwerkzeug, das auch als Implantationshilfe dienen kann, welches als minimalinvasives, insbesondere endoskopisches, Instrument, vorzugsweise Pinzetten-artig oder Zangen-artig, ausgebildet ist.

Vorteilhaft ist hier ein geringeres Trauma bei der operativen Implantation der Gehörknöchelchenprothese, da nur eine kleinere Öffnung des Trommelfells usw. notwendig wird.

Für die Anwendung der vorliegenden Erfindung in der Praxis ist auch ein Verfahren zur Implantation einer erfindungsgemäßen Gehörknöchelchenprothese der oben beschriebenen Art nützlich, welches sich dadurch auszeichnet, dass das erste Befestigungselement, insbesondere die Ankoppelbereiche innerhalb der Kopfplattenebene, Strukturen aus Memory-Metall aufweisen, und dass eine Krafteinleitung auf die Stegelemente mittels, vorzugsweise kontaktloser, insbesondere durch Lichteinstrahlung, beispielsweise Laserstrahlung, hervorgerufener, Erwärmung der Strukturen aus Memory-Metall und deren dadurch erfolgende Verformung bewirkt wird.

Damit wird insbesondere ein berührungsloser Krafteintrag in die erfindungsgemäße Gehörknöchelchenprothese, beispielsweise mittels Wärmestrahlung, ermöglicht.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

### Detaillierte Beschreibung der Erfindung anhand der Zeichnung

In der schematischen Zeichnung sind Ausführungsbeispiele der Erfindung dargestellt, welche in der nachfolgenden Beschreibung näher erläutert werden.

Es zeigen:

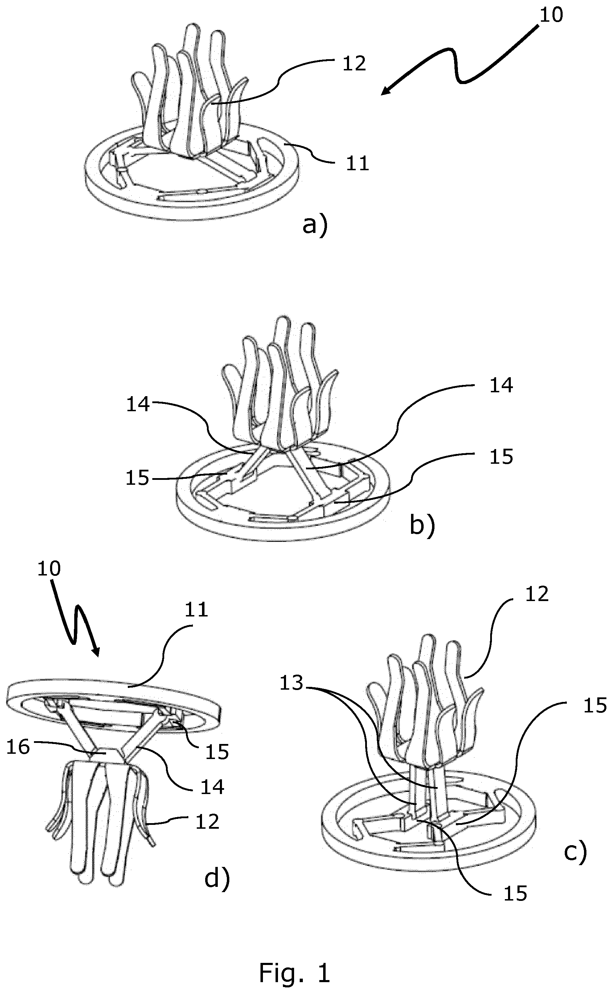

- Fig. 1: vier Abbildungen einer schematischen räumlichen Darstellung einer ersten Ausführungsform der erfindungsgemäßen Gehörknöchelchenprothese, davon drei Abbildungen in einer Sicht schräg von oben mit Blickrichtung vom zweiten zum ersten Befestigungselement, wobei die Stegelemente a) nur sehr wenig b) mittelmäßig c) nahezu voll in z-Richtung aus der Kopfplatten-Ebene ausgefahren sind,

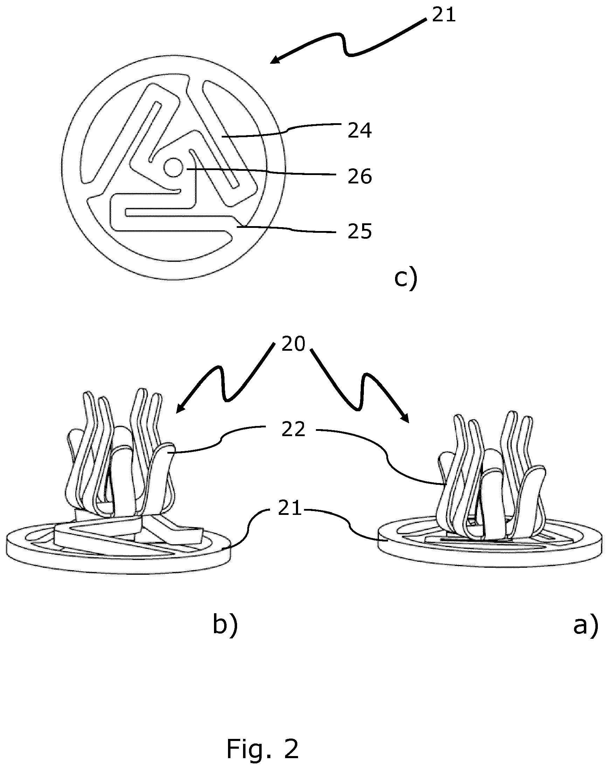

- Fig. 2: sowie eine vierte Abbildung d) mit seitlicher Sicht auf die Gehörknöchelchenprothese in einer Blickrichtung leicht schräg von unten auf die Kopfplatte; eine weitere Ausführungsform einer erfindungsgemäß gestalteten Kopfplatte, nämlich a) mit Blick leicht schräg auf die Gehörknöchelchenprothese in Blickrichtung vom zweiten zum ersten Befestigungs-element, wobei die Stegelemente vor dem Einleiten einer axialen Kraft noch vollständig in der Kopfplatten-Ebene angeordnet sind, b) wie a), jedoch mit leicht ausgefahrenen Stegelementen, sowie c) die zu dieser Ausführungsform gehörige Kopfplatte mit Blick darauf in z-Richtung von oben;

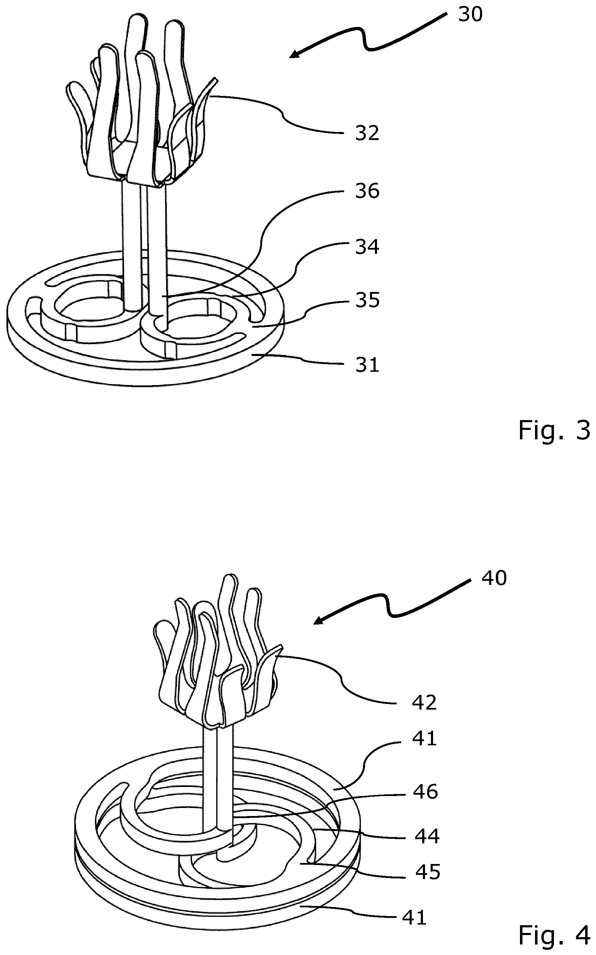

- Fig. 3: eine weitere Ausführungsform einer erfindungsgemäß gestalteten Gehörknöchelchenprothese, bei der das Verbindungselement zwischen dem ersten und zweiten Befestigungselement zwei parallele starre Schaftstücke umfasst; und
- Fig. 4: eine Ausführungsform einer erfindungsgemäßen Gehörknöchelchenprothese, bei der das erste Befestigungselement als geteilte Kopfplatte in zwei parallelen Ebenen senkrecht zur z-Achse gestaltet ist, wobei das Verbindungselement zwischen dem ersten und zweiten Befestigungselement jeweils ein in jede der beiden Kopfplattenebenen laufendes Schaftstück umfasst.

Die in den Figuren der Zeichnung schematisch dargestellten Ausführungsformen der erfindungsgemäßen **Gehörknöchelchenprothese 10; 20; 30; 40** (oder Teilen davon) weisen jeweils am einen Ende ein **erstes Befestigungselement 11; 21; 31; 41** auf, welches in Form einer ebenen Kopfplatte zur mechanischen Anlage am Trommelfell und/oder am Hammergriff ausgebildet ist. Am anderen Ende der Gehörknöchelchenprothese 10; 20; 30; 40 sitzt jeweils ein **zweites Befestigungselement 12; 22; 32; 42** zur mechanischen Verbindung der Prothese mit einem Glied oder Teilen eines Gliedes der Gehörknöchelchen-Kette oder mit dem Innenohr. Dazwischen ist ein jeweils entlang einer **Längsachse z** die beiden Befestigungselemente 11,12 bzw. 21,22 bzw. 31,32 bzw. 41,42 Schall-leitend miteinander verbindendes **Verbindungselement 13** angeordnet, welches bei manchen Ausführungsformen in Form eines -meist länglichen- Schaftes ausgeführt sein kann.

Das Verbindungselement 13 umfasst jeweils **Stegelemente 14; 24; 34; 44,** die -zumindest abschnittsweise- radial von der Längsachse z weg nach außen mehr oder weniger abgespreizt werden können und dabei die axiale Länge der Gehörknöchelchenprothese 10; 20; 30; 40 mehr oder weniger verkürzen.

Die Erfindung zeichnet sich gegenüber bekannten gattungsgemäßen Gehörknöchelchenprothesen dadurch aus, dass die Stegelemente 14; 24; 34; 44 einenends direkt in **Ankoppelbereiche 15; 25; 35; 45** des ersten Befestigungselements 11; 21; 31; 41 innerhalb der Kopfplattenebene münden und mit diesen beweglich, aber unlösbar verbunden sind,
dass sämtliche Stegelemente 14; 24; 34; 44 anderenends direkt in ein **Ankoppelelement 16; 26; 36; 46** münden und mit diesem ebenfalls beweglich, aber unlösbar verbunden sind, wobei das Ankoppelelement 16; 26; 36; 46 seinerseits anderenends mit dem zweiten Befestigungs-element 12; 22; 32; 42 starr verbunden ist,
dass die Stegelemente 14; 24; 34; 44 in einem im menschlichen Mittelohr eingesetzten in-situ-Zustand der Gehörknöchelchenprothese 10; 20; 30; 40 bei Einleitung einer Kraft auf die Stegelemente 14; 24; 34; 44 mit Kraftkomponente parallel zur Längsachse z in Richtung vom ersten Befestigungselement 11; 21; 31; 41 zum zweiten Befestigungs-element 12; 22; 32; 42 in situ jeweils abschnittsweise eine radial weiter von der Längsachse z liegende Position einnehmen und damit die axiale Funktionslänge zwischen dem ersten Befestigungselement 11; 21; 31; 41 und dem zweiten Befestigungselement 12; 22; 32; 42 in situ verkürzen, wobei die Stegelemente 14; 24; 34; 44 bei Einleitung einer Kraft mit Kraftkomponente antiparallel zur Längsachse z in Richtung vom zweiten Befestigungselement 12; 22; 32; 42 zum ersten Befestigungselement 11; 21; 31; 41 in situ jeweils abschnittsweise eine radial näher an der Längsachse z liegende Position einnehmen und damit die axiale Funktionslänge zwischen dem ersten Befestigungs-element 11; 21; 31; 41 und dem zweiten Befestigungselement 12; 22; 32; 42 in situ vergrößern,
und dass die Stegelemente 14; 24; 34; 44 ohne Einwirkung einer Kraft ihre jeweils eingestellte radiale Position zur Längsachse z in situ beibehalten.

Die drei **Ansichten a) bis c)** von **Fig. 1** stellen die Gehörknöchelchenprothese 10 in drei unterschiedlichen Betriebsstellungen dar, nämlich
a) in einem nur sehr gering ausgefahrenen Zustand der Stegelemente 14, bei dem diese noch nahezu in der Kopfplattenebene des ersten Befestigungselements 11 angeordnet sind,
b) mit mittelmäßiger Ausfahrstrecke der Stegelemente 14 und entsprechend vergrößerter Funktionslänge der Prothese zwischen dem ersten Befestigungselement 11 und dem zweiten Befestigungselement 12, sowie
c) mit voll in z-Richtung aus der Kopfplatten-Ebene ausgefahrenen Stegelementen 14.

Die vierte **Ansicht d)** von **Fig. 1** zeigt die Gehörknöchelchenprothese 10 seitlich schräg von unten mit Blickrichtung vom zweiten Befestigungselement 12 zum ersten Befestigungselement 11 in einem "halb-ausgefahrenen" Zustand der Stegelemente 14 (etwa so wie in Fig.1 b)). Hier ist das Ankoppelelement 16 gut zu erkennen.

Die beiden ersten **Ansichten a) und b)** von **Fig. 2** veranschaulichen zwei Betriebsstellungen einer weiteren Ausführungsform der erfindungsgemäßen Gehörknöchelchenprothese 20, nämlich
a) mit noch vollständig in der Kopfplatten-Ebene angeordneten Stegelementen 24 vor dem Einleiten einer axialen Kraft, und
b) mit etwas in z-Richtung ausgefahrenen, Helix-förmig gestalteten Stegelementen nach dem Einleiten einer axialen Kraft.

Die dritte **Ansicht c)** von **Fig. 2** zeigt die zu dieser Ausführungsform gehörige Kopfplatte 21 mit Draufblick von oben in z-Richtung.

In **Fig. 3** ist eine weitere Ausführungsform einer erfindungsgemäß gestalteten Gehörknöchelchenprothese 30 dargestellt, bei der das Verbindungselement zwischen dem ersten Befestigungselement 31 und dem zweiten Befestigungselement 32 zwei parallel zur z-Achse verlaufende starre Schaftstücke umfasst. Ein Ende dieser beiden Schaftstücke dient hier jeweils als Ankoppel-element 36, in welches die ringförmigen Stegelemente 34 ausgehend von ihrem jeweiligen Ankoppelbereich 35 im ersten Befestigungs-element 31 an ihrem dem zweiten Befestigungselement 32 zugewandten Ende münden.

**Fig. 4** schließlich stellt eine weitere Variante einer erfindungsgemäßen Gehörknöchelchenprothese 40 dar, bei der das erste Befestigungs-element 41 als geteilte Kopfplatte in zwei parallelen Ebenen gestaltet ist. Das Verbindungselement zwischen dem ersten Befestigungselement 41 und dem zweiten Befestigungselement 42 umfasst wiederum zwei parallel Schaftstücke, wobei aber die beiden Schaftstücke aufgrund des axialen Abstands der beiden Hälften des ersten Befestigungselements 41 voneinander unterschiedliche Längen in z-Richtung aufweisen. Auch hier dient das dem ersten Befestigungselement 41 zugewandte Ende dieser beiden Schaftstücke jeweils als Ankoppelelement 46 für die von ihrem jeweiligen Ankoppelbereich 45 im ersten Befestigungselement 41 ausgehenden Stegelemente 44. Beim zweiten Befestigungselement 42 enden dann beide Schaftstücke auf gleicher axialer Höhe.

### Bezugszeichenliste:

- 10; 20; 30; 40: Gehörknöchelchenprothese
- 11; 21; 31; 41: erstes Befestigungselement
- 12; 22; 32; 42: zweites Befestigungselement
- 13: Verbindungselement
- 14; 24; 34; 44: Stegelemente
- 15; 25; 35; 45: Ankoppelbereiche
- 16; 26; 36; 46: Ankoppelelement
- z: Längsachse

### Referenzliste

Für die Beurteilung der Patentfähigkeit in Betracht gezogene Publikationen:
[0] DE 198 45 906 A1
[00] SU 957 894 A1
[1] US 10,687,937 B2
[2] DE 42 10 235 C1; EP 0 809 982 B1; US 6,387,128 B1
[3] EP 1 181 907 B1
[4] US 2004/0162614 A1
[5] EP 1 833 424 B1
[6] EP 1 972 307 B1
[7] EP 3 311 773 B1
[8] DE 10 2007 041 539 B4
[9] EP 2 238 946 B1
[10] EP 2 601 909 B1
[11] EP 3 130 315 B1
[12] EP 1 961 400 B1

## Patentansprüche

1. Gehörknöchelchenprothese (10; 20; 30; 40), die mindestens ein Glied oder Teile eines Gliedes der Gehörknöchelchenkette ersetzt oder überbrückt, wobei die Gehörknöchelchenprothese (10; 20; 30; 40) an ihrem einen Ende ein als ebene Kopfplatte zur mechanischen Anlage am Trommelfell und/oder am Hammergriff ausgebildetes erstes Befestigungselement (11; 21; 31; 41) und an ihrem anderen Ende ein zweites Befestigungselement (12; 22; 32; 42) zur mechanischen Verbindung mit einem Glied oder Teilen eines Gliedes der Gehörknöchelchen-Kette oder mit dem Innenohr sowie ein entlang einer Längsachse (z) die beiden Befestigungselemente (11,12; 21,22; 31,32; 41,42) Schall leitend miteinander verbindendes Verbindungselement (13) umfasst, wobei das Verbindungselement (13) Stegelemente (14; 24; 34; 44) aufweist, die -zumindest abschnittsweise- radial von der Längsachse (z) weg nach außen abgespreizt werden können und dabei die axiale Länge der Gehörknöchelchenprothese (10; 20; 30; 40) verkürzen,wobei die Stegelemente (14; 24; 34; 44) einenends direkt in Ankoppelbereiche (15; 25; 35; 45) des ersten Befestigungselements (11; 21; 31; 41) innerhalb der Kopfplattenebene münden und mit diesen beweglich, aber unlösbar verbunden sind,
wobei sämtliche Stegelemente (14; 24; 34; 44) anderenends direkt in ein Ankoppelelement (16; 26; 36; 46) münden und mit diesem ebenfalls beweglich, aber unlösbar verbunden sind, wobei das Ankoppelelement (16; 26; 36; 46) seinerseits anderenends mit dem zweiten Befestigungselement (12; 22; 32; 42) starr verbunden ist,
wobei die Stegelemente (14; 24; 34; 44) derart ausgebildet sind, dass sie in einem im menschlichen Mittelohr eingesetzten in-situ-Zustand der Gehörknöchelchenprothese (10; 20; 30; 40) bei Einleitung einer Kraft auf die Stegelemente (14; 24; 34; 44) mit Kraftkomponente parallel zur Längsachse (z) in Richtung vom ersten Befestigungselement (11; 21; 31; 41) zum zweiten Befestigungselement (12; 22; 32; 42) in situ jeweils abschnittsweise eine radial weiter von der Längsachse (z) liegende Position einnehmen und damit die axiale Funktionslänge zwischen dem ersten Befestigungselement (11; 21; 31; 41) und dem zweiten Befestigungselement (12; 22; 32; 42) in situ verkürzen, wobei die Stegelemente (14; 24; 34; 44) bei Einleitung einer Kraft mit Kraftkomponente antiparallel zur Längsachse (z) in Richtung vom zweiten Befestigungselement (12; 22; 32; 42) zum ersten Befestigungselement (11; 21; 31; 41) in situ jeweils abschnittsweise eine radial näher an der Längsachse (z) liegende Position einnehmen und damit die axiale Funktionslänge zwischen dem ersten Befestigungselement (11; 21; 31; 41) und dem zweiten Befestigungselement (12; 22; 32; 42) in situ vergrößern,
und wobei die Stegelemente (14; 24; 34; 44) ohne Einwirkung einer Kraft ihre jeweils eingestellte radiale Position zur Längsachse (z) in situ beibehalten,
**dadurch gekennzeichnet,**
**dass** die Ankoppelbereiche (15; 25; 35; 45), die Stegelemente (14; 24; 34; 44) sowie das Ankoppelelement (16; 26; 36; 46) vor dem erstmaligen Einleiten einer Kraft auf die Stegelemente (14; 24; 34; 44) mit Kraftkomponente parallel oder antiparallel zur Längsachse (z) flach innerhalb der Kopfplattenebene des ersten Befestigungselements (11; 21; 31; 41) angeordnet sind;
**dass** die Stegelemente (14; 24; 34; 44) zwischen ihrem jeweiligen Ankoppelbereich (15; 25; 35; 45) und dem Ankoppelelement (16; 26; 36; 46) kurvenförmig und/oder mäanderförmig und/oder im Zickzack verlaufen;
**dass** das Ankoppelelement (16; 26; 36; 46) in seiner Ausgangsposition innerhalb der Kopfplattenebene zentrisch im ersten Befestigungselement (11; 21; 31; 41) angeordnet ist;
**dass** radial äußere, freie Endabschnitte der Stegelemente (14; 24; 34; 44) atraumatische, nicht-spitzzulaufende, bezüglich der Längsachse radial äußere, quer zur Richtung der Längsachse (z) ausgedehnte, freie Endkanten aufweisen oder als atraumatische Flächen ausgebildet sind;
und **dass** Arretierungseinrichtungen vorhanden sind, welche beim Einleiten einer axialen Kraft auf die Stegelemente (14; 24; 34; 44) bei einer oder mehreren axialen Längen des Verbindungselements (13) jeweils einen mechanischen Widerstand hervorrufen.

2. Gehörknöchelchenprothese nach Anspruch 1, **dadurch gekennzeichnet, dass** die Stegelemente (14; 24; 34; 44) derart ausgebildet sind, dass ihre jeweils eingestellte radiale Position zur Längsachse (z) in situ durch Einleiten einer entsprechenden Kraft reversibel veränderbar ist.

3. Gehörknöchelchenprothese nach einem der vorhergehenden Ansprüche, **dadurch gekennzeichnet, dass** die innerhalb der Kopfplattenebene des ersten Befestigungselements (11; 21; 31; 41) angeordneten Ankoppelbereiche (15; 25; 35; 45) geometrisch so gestaltet sind, dass über sie die Einleitung einer Kraft auf die Stegelemente (14; 24; 34; 44) mit Kraftkomponente parallel oder antiparallel zur Längsachse (z) mittels eines Einstellwerkzeugs in situ erfolgen kann.

4. Gehörknöchelchenprothese nach einem der vorhergehenden Ansprüche, **dadurch gekennzeichnet, dass** die Verbindungsstellen der Stegelemente (14; 24; 34; 44) mit den Ankoppelbereichen (15; 25; 35; 45) des ersten Befestigungselements (11; 21; 31; 41) sowie die Verbindungsstellen der Stegelemente (14; 24; 34; 44) mit dem Ankoppelelement (16; 26; 36; 46) jeweils als mechanische Gelenke oder Biegestellen ausgebildet sind.

5. Gehörknöchelchenprothese nach einem der Ansprüche 1 bis 4, **dadurch gekennzeichnet, dass** die Stegelemente (14; 24; 34; 44) zumindest abschnittsweise, vorzugsweise vollständig mechanisch starr ausgebildet sind.

6. Gehörknöchelchenprothese nach Anspruch 5, **dadurch gekennzeichnet, dass** zwischen den mechanisch starren Abschnitten der Stegelemente (14; 24; 34; 44) integrierte mechanische Gelenke oder Biegestellen ausgebildet sind.

7. Gehörknöchelchenprothese nach einem der Ansprüche 1 bis 4, **dadurch gekennzeichnet, dass** die Stegelemente (14; 24; 34; 44) zumindest abschnittsweise aus einem plastischen, flexiblen Material ausgebildet sind, und dass das plastische, flexible Material der Stegelemente (14; 24; 34; 44) insbesondere eine Elastizität ≥ 1%, vorzugsweise eine Elastizität ≥ 2%, aufweist.

8. Gehörknöchelchenprothese nach Anspruch 7, **dadurch gekennzeichnet, dass** das plastische, flexible Material der Stegelemente (14; 24; 34; 44) hochelastisches Material, vorzugsweise amorphes Metall, insbesondere basierend auf Nickel, Eisen, Kobalt oder Zirkonium, und/oder eine Nickel-Titan-Legierung und/oder Memory-Metall, enthält.

9. Gehörknöchelchenprothese nach Anspruch 7, **dadurch gekennzeichnet, dass** das plastische, flexible Material der Stegelemente (14; 24; 34; 44) hochelastischen Kunststoff, insbesondere hochfestes elastisches Polymer, und/oder elastische Keramik enthält.

10. Gehörknöchelchenprothese nach einem der vorhergehenden Ansprüche, **dadurch gekennzeichnet, dass** das Ankoppelelement (16; 26; 36; 46) ringförmig aufgebaut ist.

11. System umfassend eine Gehörknöchelchenprothese (10; 20; 30; 40) nach einem der vorhergehenden Ansprüche sowie ein Einstellwerkzeug, **dadurch gekennzeichnet, dass** das Einstellwerkzeug als minimalinvasives, insbesondere endoskopisches, Instrument, vorzugsweise Pinzetten-artig oder Zangen-artig, ausgebildet ist.

## Claims

1. An ossicular prosthesis (10; 20; 30; 40), which replaces or bridges at least one member or parts of a member of the ossicular chain, the ossicular prosthesis (10; 20; 30; 40) comprising, at one end, a first fastening element (11; 21; 31; 41) formed as a flat head plate for mechanical contact with the tympanic membrane and/or with the handle of malleus, and, at its other end, a second fastening element (12; 22; 32; 42) for mechanical connection to a member or parts of a member of the ossicular chain or to the inner ear, as well as a connecting element (13) that connects the two fastening elements (11, 12; 21, 22; 31, 32; 41, 42) to one another, in a sound-conducting manner, along a longitudinal axis (z),
wherein the connecting element (13) has rib elements (14; 24; 34; 44), which can be spread outwards, at least in sections, radially away from the longitudinal axis (z) and in the process shorten the axial length of the ossicular prosthesis (10; 20; 30; 40), wherein the rib elements (14; 24; 34; 44) lead at one end directly into coupling regions (15; 25; 35; 45) of the first fastening element (11; 21; 31; 41) within the head plate plane and are movably, but non-detachably, connected thereto,
wherein all the rib elements (14; 24; 34; 44) lead directly, at the other end, into a coupling element (16; 26; 36; 46) and are also movably, but non-detachably, connected thereto, the coupling element (16; 26; 36; 46) in turn being rigidly connected at the other end to the second fastening element (12; 22; 32; 42), wherein the rib elements (14; 24; 34; 44) are designed in such a way that, in an in-situ state of the ossicular prosthesis (10; 20; 30; 40) inserted in a human middle ear, when a force is applied to the rib elements (14; 24; 34; 44) with the force component in parallel with the longitudinal axis (z) in the direction from the first fastening element (11; 21; 31; 41) to the second fastening element (12; 22; 32; 42) in each case sectionally assume, in situ, a position located radially further from the longitudinal axis (z), and therefore shorten, in situ, the axial functional length between the first fastening element (11; 21; 31; 41) and the second fastening element (12; 22; 32; 42), wherein the rib elements (14; 24; 34; 44), with the introduction of a force with a force component antiparallel with respect to the longitudinal axis (z), in the direction from the second fastening element (12; 22; 32; 42) to the first fastening element (11; 21; 31; 41) in each case sectionally assuming, in situ, a position located radially closer to the longitudinal axis (z), and therefore increasing, in situ, the axial functional length between the first fastening element (11; 21; 31; 41) and the second fastening element (12; 22; 32; 42),
and wherein the rib elements (14, 24; 34; 44) retain, in situ, their respective adjusted radial position with respect to the longitudinal axis (z), when no force acts,
**characterized in that**
the coupling regions (15; 25; 35; 45), the rib elements (14; 24; 34; 44) and the coupling element (16; 26; 36; 46) are arranged flat within the head plate plane of the first fastening element (11; 21; 31; 41) before the first application of a force to the rib elements (14; 24; 34; 44) with a force component in parallel or antiparallel with respect to the longitudinal axis (z),
**in that** the rib elements (14; 24; 34; 44) extend between their respective coupling region (15; 25; 35; 45) and the coupling element (16; 26; 36; 46) in a curved and/or meandering and/or zigzag manner,
**in that** the coupling element (16; 26; 36; 46) is arranged centrally in the first fastening element (11; 21; 31; 41) in its initial position within the head plate plane,
**in that** radially outer free end portions of the rib elements (14; 24; 34; 44) have atraumatic, non-tapering free end edges which are radially outward with respect to the longitudinal axis and extend transversely to the direction of the longitudinal axis (z), or are designed as atraumatic surfaces,
and **in that** locking devices are present which, when an axial force is applied to the rib elements (14; 24; 34; 44), each cause a mechanical resistance at one or more axial lengths of the connecting element (13).

2. The ossicular prosthesis according to claim 1, **characterized in that** the rib elements (14; 24; 34; 44) are designed such that their respective adjusted radial position relative to the longitudinal axis (z) can be reversibly changed in situ by introducing a corresponding force.

3. The ossicular prosthesis according to any of the preceding claims, **characterized in that** the coupling regions (15; 25; 35; 45) arranged within the head plate plane of the first fastening element (11; 21; 31; 41) are geometrically designed such that they can be used to apply a force to the rib elements (14; 24; 34; 44) with a force component in parallel or antiparallel with respect to the longitudinal axis (z) by means of an adjusting tool in situ.

4. The ossicular prosthesis according to any of the preceding claims, **characterized in that** the connection points of the rib elements (14; 24; 34; 44) with the coupling regions (15; 25; 35; 45) of the first fastening element (11; 21; 31; 41) and the connection points of the rib elements (14; 24; 34; 44) with the coupling element (16; 26; 36; 46) are each designed as mechanical joints or bending points.

5. The ossicular prosthesis according to any of claims 1 to 4, **characterized in that** the rib elements (14; 24; 34; 44) are designed so as to be mechanically rigid at least sectionally, preferably completely.

6. The ossicular prosthesis according to claim 5, **characterized in that** integrated mechanical joints or bending points are formed between the mechanically rigid portions of the rib elements (14; 24; 34; 44).

7. The ossicular prosthesis according to any of claims 1 to 4, **characterized in that** the rib elements (14; 24; 34; 44) are formed at least sectionally from a plastic, flexible material, and that the plastic, flexible material of the rib elements (14; 24; 34; 44) in particular has an elasticity of ≥ 1%, preferably an elasticity of ≥ 2%.

8. The ossicular prosthesis according to claim 7, **characterized in that** the plastic, flexible material of the rib elements (14; 24; 34; 44) contains highly elastic material, preferably amorphous metal, in particular based on nickel, iron, cobalt or zirconium, and/or a nickel-titanium alloy and/or memory metal.

9. The ossicular prosthesis according to claim 7, **characterized in that** the plastic, flexible material of the rib elements (14; 24; 34; 44) contains a highly elastic plastic, in particular a high-strength elastic polymer, and/or elastic ceramic.

10. The ossicular prosthesis according to any of the preceding claims, **characterized in that** the coupling element (16; 26; 36; 46) has a ring-shaped design.

11. A system comprising an ossicular prosthesis (10; 20; 30; 40) according to any of the preceding claims, and an adjusting tool, **characterized in that** the adjusting tool is designed as a minimally invasive, in particular endoscopic, instrument, preferably tweezer-like or pincer-like.

## Revendications

1. Prothèse d'osselets (10 ; 20 ; 30 ; 40) qui remplace ou ponte au moins un membre ou des parties d'un membre de la chaîne des osselets, dans laquelle la prothèse d'osselets (10 ; 20 ; 30 ; 40) comprend à l'une de ses extrémités un premier élément de fixation (11 ; 21 ; 31 ; 41) réalisé sous forme de plaque de tête plane pour l'appui mécanique sur le tympan et/ou sur le manche du marteau et, à son autre extrémité, un second élément de fixation (12 ; 22 ; 32 ; 42) pour la liaison mécanique avec un membre ou des parties d'un membre de la chaîne des osselets ou avec l'oreille interne, ainsi qu'un élément de liaison (13) reliant les deux éléments de fixation (11, 12 ; 21, 22 ; 31, 32 ; 41, 42) l'un à l'autre le long d'un axe longitudinal (z) de manière à conduire le bruit,
dans laquelle l'élément de liaison (13) présente des éléments formant entretoises (14 ; 24 ; 34 ; 44) qui peuvent être écartés, au moins dans certaines sections, radialement vers l'extérieur en s'éloignant de l'axe longitudinal (z) et qui permettent ainsi de réduire la longueur axiale de la prothèse d'osselets (10 ; 20 ; 30 ; 40), dans laquelle les éléments formant entretoises (14 ; 24 ; 34 ; 44) débouchent à une extrémité directement dans des zones d'accouplement (15 ; 25 ; 35 ; 45) du premier élément de fixation (11 ; 21 ; 31 ; 41) à l'intérieur du plan de plaque de tête et sont reliés à celles-ci de manière à être mobiles, mais indissociables,
dans laquelle tous les éléments formant entretoises (14 ; 24 ; 34 ; 44) débouchent à l'autre extrémité directement dans un élément d'accouplement (16 ; 26 ; 36 ; 46) et sont reliés à celui-ci également de manière à être mobiles, mais indissociables, dans laquelle l'élément d'accouplement (16 ; 26 ; 36 ; 46) est pour sa part relié rigidement à l'autre extrémité au second élément de fixation (12 ; 22 ; 32 ; 42),
dans laquelle les éléments formant entretoises (14 ; 24 ; 34 ; 44) sont conçus de telle sorte que, dans un état *in situ* de la prothèse d'osselets (10 ; 20 ; 30 ; 40) utilisée dans l'oreille moyenne humaine, lors de l'application d'une force sur les éléments formant entretoises (14 ; 24 ; 34 ; 44) avec une composante de force parallèle à l'axe longitudinal (z) dans une direction du premier élément de fixation (11 ; 21 ; 31 ; 41) vers le second élément de fixation (12 ; 22 ; 32 ; 42), lesdits éléments formant entretoises *in situ* prennent, respectivement dans certaines sections, une position située radialement plus loin de l'axe longitudinal (z) et raccourcissent ainsi la longueur fonctionnelle axiale entre le premier élément de fixation (11 ; 21 ; 31 ; 41) et le second élément de fixation (12 ; 22 ; 32 ; 42) *in situ,* dans laquelle les éléments formant entretoises (14 ; 24 ; 34 ; 44), lors de l'application d'une force avec une composante de force antiparallèle à l'axe longitudinal (z) dans une direction du deuxième élément de fixation (12 ; 22 ; 32 ; 42) vers le premier élément de fixation (11 ; 21 ; 31 ; 41), prennent *in situ,* respectivement dans certaines sections, une position radialement plus proche de l'axe longitudinal (z) et augmentent ainsi la longueur fonctionnelle axiale entre le premier élément de fixation (11 ; 21 ; 31 ; 41) et le second élément de fixation (12 ; 22 ; 32 ; 42) *in situ,*
et dans laquelle les éléments formant entretoises (14 ; 24 ; 34 ; 44) conservent *in situ* leur position radiale respectivement réglée par rapport à l'axe longitudinal (z) sans l'action d'une force,
**caractérisée en ce**
**que** les zones d'accouplement (15 ; 25 ; 35 ; 45), les éléments formant entretoises (14 ; 24 ; 34 ; 44) ainsi que l'élément d'accouplement (16 ; 26 ; 36 ; 46) sont disposés à plat à l'intérieur du plan de plaque de tête du premier élément de fixation (11 ; 21 ; 31 ; 41) avant l'application initiale d'une force sur les éléments formant entretoises (14 ; 24 ; 34 ; 44) avec une composante de force parallèle ou antiparallèle à l'axe longitudinal (z);
**que** les éléments formant entretoises (14 ; 24 ; 34 ; 44) s'étendent en forme de courbe et/ou de méandres et/ou en zigzag entre leur zone d'accouplement (15 ; 25 ; 35 ; 45) respective et l'élément d'accouplement (16 ; 26 ; 36 ; 46);
**que** l'élément d'accouplement (16 ; 26 ; 36 ; 46) est disposé, dans sa position de départ, à l'intérieur du plan de plaque de tête de manière à être centré dans le premier élément de fixation (11 ; 21 ; 31 ; 41);
**que** des sections d'extrémité libres radialement extérieures des éléments formant entretoises (14 ; 24 ; 34 ; 44) présentent des arêtes d'extrémité libres radialement extérieures, non pointues, atraumatiques, radialement extérieures par rapport à l'axe longitudinal et s'étendant transversalement à la direction de l'axe longitudinal (z), ou sont réalisées sous forme de surfaces atraumatiques;
et **que** des dispositifs d'arrêt sont prévus, lesquels, lors de l'application d'une force axiale sur les éléments formant entretoises (14 ; 24 ; 34 ; 44), provoquent respectivement une résistance mécanique pour une ou plusieurs longueurs axiales de l'élément de liaison (13).

2. Prothèse d'osselets selon la revendication 1, **caractérisée en ce que** les éléments formant entretoises (14 ; 24 ; 34 ; 44) sont conçus de telle sorte que leur position radiale respectivement réglée par rapport à l'axe longitudinal (z) peut être modifiée *in situ* de manière réversible par l'application d'une force correspondante.

3. Prothèse d'osselets selon l'une des revendications précédentes, **caractérisée en ce que** les zones d'accouplement (15 ; 25 ; 35 ; 45) disposées à l'intérieur du plan de plaque de tête du premier élément de fixation (11 ; 21 ; 31 ; 41) sont conçues géométriquement de sorte que, par l'intermédiaire de celles-ci, l'application d'une force sur les éléments formant entretoises (14 ; 24 ; 34 ; 44) avec une composante de force parallèle ou antiparallèle à l'axe longitudinal (z) peut être effectuée *in situ* au moyen d'un outil de réglage.

4. Prothèse d'osselets selon l'une des revendications précédentes, **caractérisée en ce que** les points de liaison des éléments formant entretoises (14 ; 24 ; 34 ; 44) avec les zones d'accouplement (15 ; 25 ; 35 ; 45) du premier élément de fixation (11 ; 21 ; 31 ; 41) ainsi que les points de liaison des éléments formant entretoises (14 ; 24 ; 34 ; 44) avec l'élément d'accouplement (16 ; 26 ; 36 ; 46) sont respectivement réalisés comme des articulations mécaniques ou des points de flexion.

5. Prothèse d'osselets selon l'une des revendications 1 à 4, **caractérisée en ce que** les éléments formant entretoises (14 ; 24 ; 34 ; 44) sont **réalisés** de manière à être mécaniquement rigides au moins dans certaines sections, de préférence entièrement.

6. Prothèse d'osselets selon la revendication 5, **caractérisée en ce que** des articulations mécaniques intégrées ou des points de flexion intégrés sont réalisés entre les sections mécaniquement rigides des éléments formant entretoises (14 ; 24 ; 34 ; 44).

7. Prothèse d'osselets selon l'une des revendications 1 à 4, **caractérisée en ce que** les éléments formant entretoises (14 ; 24 ; 34 ; 44) sont réalisés, au moins dans certaines sections, en un matériau plastique et flexible, et que le matériau plastique et flexible des éléments formant entretoises (14 ; 24 ; 34 ; 44) présente en particulier une élasticité ≥ 1 %, de préférence une élasticité ≥ 2 %.

8. Prothèse d'osselets selon la revendication 7, **caractérisée en ce que** le matériau plastique et flexible des éléments formant entretoises (14 ; 24 ; 34 ; 44) contient un matériau hautement élastique, de préférence un métal amorphe, en particulier à base de nickel, fer, cobalt ou zirconium, et/ou un alliage de nickel et de titane et/ou un métal à mémoire de forme.

9. Prothèse d'osselets selon la revendication 7, **caractérisée en ce que** le matériau plastique et flexible des éléments formant entretoises (14 ; 24 ; 34 ; 44) contient une matière plastique hautement élastique, en particulier un polymère élastique hautement résistant, et/ou une céramique élastique.

10. Prothèse d'osselets selon l'une des revendications précédentes, **caractérisée en ce que** l'élément d'accouplement (16, 26, 36, 46) est conçu en forme d'anneau.

11. Système comprenant une prothèse d'osselets (10 ; 20 ; 30 ; 40) selon l'une des revendications précédentes ainsi qu'un outil de réglage,
**caractérisé en ce que** l'outil de réglage est réalisé comme un instrument mini-invasif, en particulier endoscopique, de préférence à la manière d'une pincette ou d'une pince.
